# EUROPEAN PATENT APPLICATION

(11) **EP 4 467 170 A1**
(43) Date of publication of application: **27.11.2024**
(21) Application number: 23740349.8
(22) Date of filing: 16.01.2023
(51) Int. Cl.: A61M 1/00, A61M 25/00

(54) **DRAINAGE TUBE**

(30) Priority: 17.01.2022 JP 2022004924
(71) Applicant: Tohoku University, Sendai-shi, Miyagi 980-8577 (JP); Okuzono, Toru, Sendai-shi, Miyagi 981-0942 (JP)
(72) Inventor: MIYAMOTO, Koichiro, Sendai-shi, Miyagi 980-8577 (JP); OKUZONO, Toru, Sendai-shi, Miyagi 981-0942 (JP)
(74) Representative: Plasseraud IP
(86) International application number: PCT/JP2023/000958
(87) International publication number: WO 2023/136341

(57) **Abstract**

A drainage tube for discharging drainage such as pus accumulated in a gallbladder (11) in a body to outside of the gallbladder (11), the drainage tube (1) including: a front-stage tube (2) to be inserted into the gallbladder (11); and a rear-stage tube (3) joined to an end portion on a side opposite to an end portion to be inserted into the gallbladder (11) between both ends of the front-stage tube (2), in which the front-stage tube (2) and the rear-stage tube (3) are joined to each other by fitting an inner surface of the rear-stage tube (3) to an outer surface of the front-stage tube (2). An extrusion member that is used to separate the front-stage tube (2) from the rear-stage tube (3), and that extrudes, from the rear-stage tube (3), an end portion on a side opposite to an end portion to be inserted into a tissue between both the ends of the front-stage tube (2) is provided.

## Description

### Technical Field

The present invention relates to a drainage tube.

The present application claims priority to Japanese Patent Application No. 2022-004924 filed in Japan on January 17, 2022, and the contents of which are incorporated herein.

### Background Art

In the related art, a biliary tract drainage tube used as a pipe that discharges bile to outside of a body or a duodenum is known. Here, the bile is a liquid produced by a liver, and flows into the duodenum through the bile duct, the gallbladder, and a common bile duct. The bile has a function of making digestive enzyme of a pancreatic juice active by being combined with the pancreatic juice in the duodenum to decompose fat and proteins and facilitate absorption from an intestine. Further, since fatty acid produced when the fat is decomposed is difficult to absorb, the bile also has a function of changing the fatty acid into an easy-to-absorb shape.

Incidentally, there is a symptom (obstructive jaundice) in which the biliary tract is obstructed by a gallstone or a tumor and the bile stops flowing. When biliary obstruction or the like occurs, flow of the bile may be stagnant, but the liver continues to produce the bile even if the flow of the bile is stagnant. When an amount of the bile produced by the liver is larger than an amount of the bile that flows into the duodenum, the bile is stored in the gallbladder. However, when the gallbladder is full, a part of the bile produced by the liver is accumulated in the liver. If such bile flows into the duodenum, the bile implements the function described above, but a tissue of the liver may be damaged when the bile is accumulated in the liver. There is also a possibility of causing hepatic cirrhosis. Therefore, when the biliary obstruction occurs, biliary decompression (drainage) for improving the flow of the bile is necessary.

When acute cholecystitis develops and surgery cannot be performed, gallbladder drainage that discharges the bile and pus from the gallbladder is necessary. The gallbladder drainage includes surgical percutaneous transhepatic drainage and endoscopic gallbladder drainage. In the endoscopic gallbladder drainage, a tube or a stent is disposed in a form of passing through a gallbladder wall from a stomach wall or a duodenum wall to form a flow path of the bile.

There is a method in which when performing drainage on the biliary tract, an endoscope is used and a tube is disposed in the biliary tract to form the flow path of the bile. As a drainage tube used when performing the drainage on the biliary tract, there is a drainage tube including, for example, a long external fistula tube, and a short internal fistula tube detachably provided at a tip end of the external fistula tube as shown in Patent Literature 1. In this case, a rear-stage tube and a front-stage tube are coupled by winding a falling-off prevention portion formed of a material that can be melted in a body. Alternatively, fixing is performed by a sheet-shaped member.

### Citation List

### Patent Literature

PTL 1: WO 2015/133333

### Summary of Invention

### Technical Problem

In the drainage tube disclosed in Patent Literature 1 as described above, when the falling-off prevention portion or the sheet-shaped member, which is formed of the material that can be melted in the body, is used, the long external fistula tube (hereinafter, referred to as the "rear-stage tube") and the short internal fistula tube (hereinafter, referred to as the "front-stage tube") cannot be separated from each other at arbitrary timing.

For example, pus may be assumed as drainage accumulated in an organ, but since viscosity of the pus is not always constant, and a treatment of diluting the pus is once required, it is required to be able to control a timing of separation between the rear-stage tube and the front-stage tube, and there is room for improvement in this regard.

Furthermore, when the falling-off prevention portion or the sheet-shaped member, which is formed of the material that can be melted in the body, is used, there is also a problem that the number of members is increased, and therefore the structure of the drainage tube becomes complicated.

The invention has been made in view of the above-described problems, and an object thereof is to provide a drainage tube that can control a timing of separation between a rear-stage tube and a front-stage tube while adopting a simple structure.

### Solution to Problem

In order to solve the above problems and achieve the object, the invention adopts the following aspects.
(1) A drainage tube according to a first aspect of the invention is a drainage tube for discharging drainage such as pus accumulated in a tissue in a body to outside of the tissue, the drainage tube including: a front-stage tube to be inserted into the tissue; and a rear-stage tube joined to an end portion on a side opposite to an end portion to be inserted into the tissue between both ends of the front-stage tube, in which the front-stage tube and the rear-stage tube are joined to each other by fitting an inner surface of the rear-stage tube to an outer surface of the front-stage tube.
(2) In the drainage tube according to the above (1), it is preferable that an extrusion member that is used to separate the front-stage tube from the rear-stage tube, and that extrudes, from the rear-stage tube, the end portion on the side opposite to the end portion to be inserted into the tissue between both the ends of the front-stage tube is provided.
(3) In the drainage tube according to the above (2), it is preferable that the extrusion member is provided to be capable of pressing the front-stage tube in a state of being inserted through inside of the rear-stage tube.
(4) In the drainage tube according to the above (2), it is preferable that the extrusion member is provided so that the rear-stage tube is movable in a direction away from the front-stage tube in a state where the extrusion member is locked to a peripheral surface of the front-stage tube.
(5) In the drainage tube according to any one of the above (1) to (4), an energization wire having an outer diameter smaller than an inner diameter of the front-stage tube and configured to form a through hole in the tissue with a tip end thereof by causing a high-frequency current to flow may be inserted through the front-stage tube and the rear-stage tube.
(6) In the drainage tube according to any one of the above (1) to (4), it is preferable that the tissue in the body is one or more tissues selected from a gallbladder, a bile duct, an abscess, and a gastrointestinal tract.
(7) In the drainage tube according to any one of the above (1) to (4), it is preferable that the rear-stage tube is led out to outside of the body, and after a diluent is injected from outside of the body into the tissue in the body via the rear-stage tube and the front-stage tube and is mixed with the drainage, a mixture of the drainage and the diluent is led out to the outside of the body via the front-stage tube and the rear-stage tube.
(8) In the drainage tube according to the above (7), after the injection of the diluent and the leading out of the mixture to the outside of the body are performed one time or more, the rear-stage tube may be separated from the front-stage tube.

### Advantageous Effects of Invention

According to a drainage tube in the aspects of the invention, it is possible to control a timing of separation between a rear-stage tube and a front-stage tube.

### Brief Description of Drawings

[FIG. 1] FIG. 1 is a side sectional view showing an overall view of a drainage tube according to the present embodiment.
[FIG. 2] FIG. 2 is a side sectional view showing a joined portion between a rear-stage tube and a front-stage tube.
[FIG. 3A] FIG. 3A is a side view showing a shape of the front-stage tube.
[FIG. 3B] FIG. 3B is a side view showing a shape of the front-stage tube.
[FIG. 3C] FIG. 3C is a side view showing a shape of the front-stage tube.
[FIG. 3D] FIG. 3D is a side view showing a shape of the front-stage tube.
[FIG. 3E] FIG. 3E is a side view showing a shape of the front-stage tube.
[FIG. 3F] FIG. 3F is a side view showing a shape of the front-stage tube.
[FIG. 4] FIG. 4 is a side view showing a configuration of an energization wire.
[FIG. 5] FIG. 5 is a side view showing a configuration of an extrusion member.
[FIG. 6] FIG. 6 is a side sectional view showing a state where the drainage tube does not protrude from an endoscope.
[FIG. 7] FIG. 7 is a side sectional view showing a state where the front-stage tube is caused to protrude from a tip end portion of the endoscope by an operation portion in FIG. 6.
[FIG. 8] FIG. 8 is a side sectional view showing a state where a pass-through portion is provided in an organ wall by inserting the energization wire through the drainage tube.
[FIG. 9A] FIG. 9A is a side sectional view of main parts showing a work procedure of inserting through the energization wire to provide the pass-through portion in the organ wall.
[FIG. 9B] FIG. 9B is the side sectional view of the main parts showing the work procedure of inserting through the energization wire to provide the pass-through portion in the organ wall.
[FIG. 9C] FIG. 9C is the side sectional view of the main parts showing the work procedure of inserting through the energization wire to provide the pass-through portion in the organ wall.
[FIG. 10] FIG. 10 is a side sectional view showing a state where an injector is used to inject a cleaning liquid into a gallbladder.
[FIG. 11] FIG. 11 is a side sectional view showing a state where the extrusion member is inserted into the rear-stage tube.
[FIG. 12] FIG. 12 is a side sectional view showing a state where a mixed liquid obtained by mixing the cleaning liquid with bile in the gallbladder is discharged to outside of a body.
[FIG. 13A] FIG. 13A is a side sectional view showing a work procedure of separating the front-stage tube from the rear-stage tube by the extrusion member.
[FIG. 13B] FIG. 13B is a side sectional view showing the work procedure of separating the front-stage tube from the rear-stage tube by the extrusion member.
[FIG. 13C] FIG. 13C is a side sectional view showing the work procedure of separating the front-stage tube from the rear-stage tube by the extrusion member.
[FIG. 14A] FIG. 14A is a side sectional view showing a work procedure of separating the front-stage tube from the rear-stage tube by an extrusion member according to a modification.
[FIG. 14B] FIG. 14B is a side sectional view showing the work procedure of separating the front-stage tube from the rear-stage tube by the extrusion member according to the modification.

### Description of Embodiments

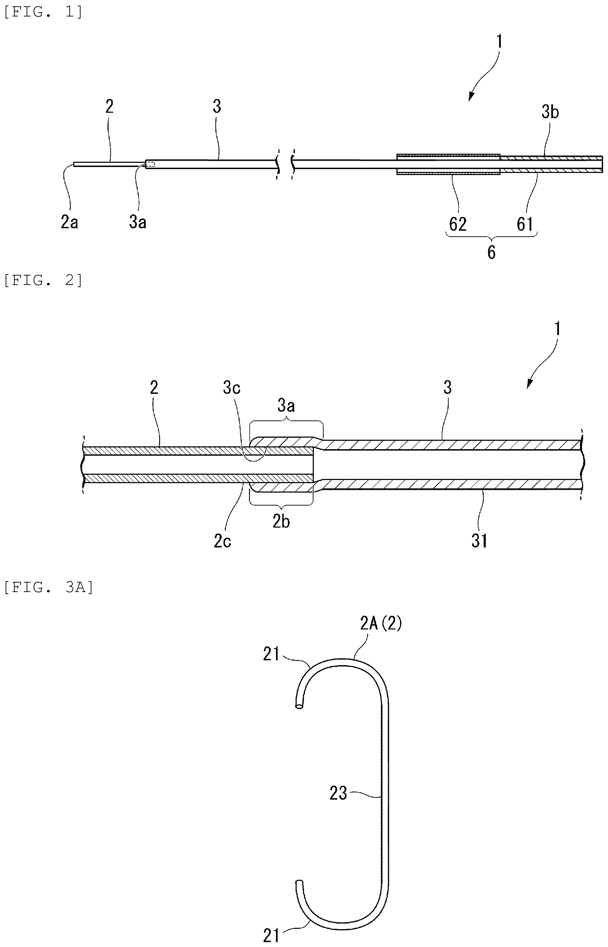

An example of a drainage tube according to an embodiment of the invention will be described with reference to FIGS. 1 to 13. FIG. 1 is an overall view of a drainage tube 1.

### [Drainage Tube 1]

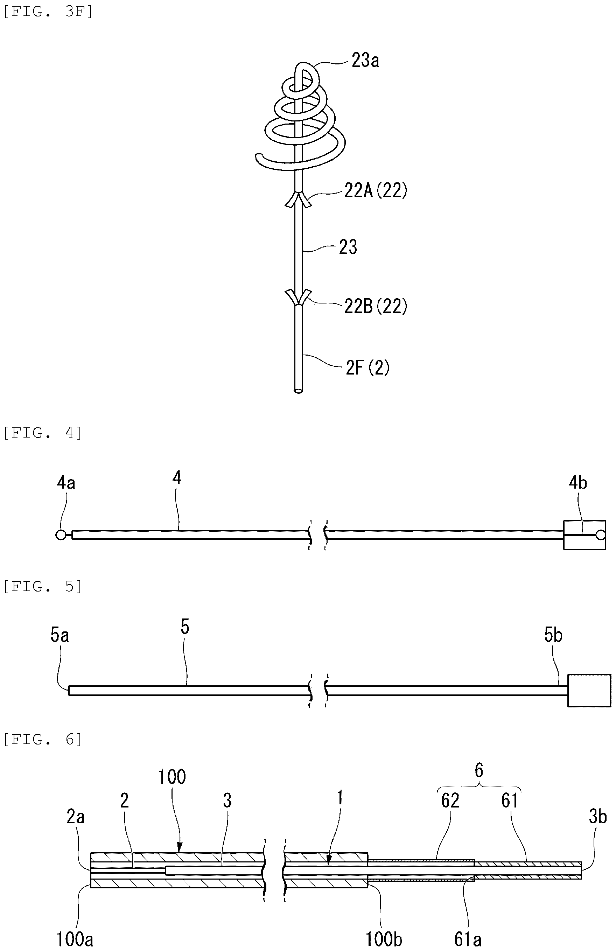

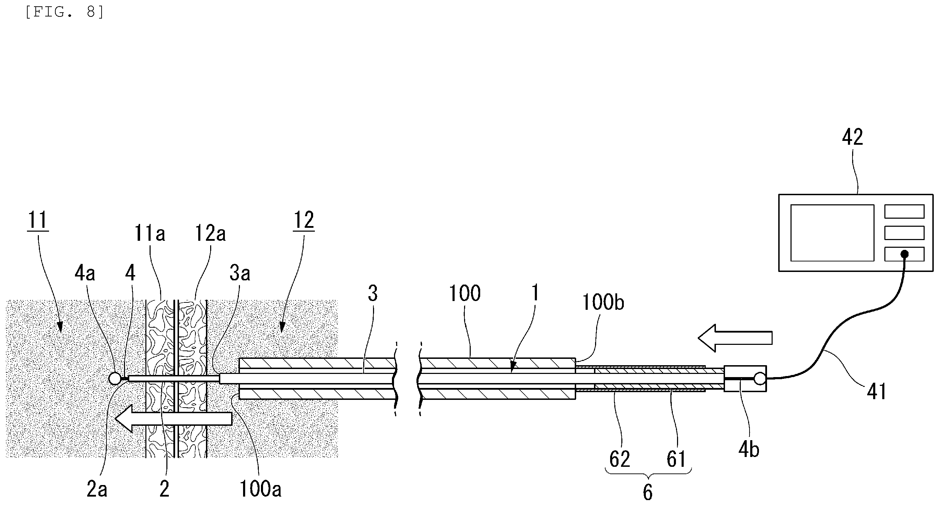

As shown in FIG. 1, the drainage tube 1 is used to discharge drainage such as pus accumulated in one or more tissues in a body selected from, for example, a gallbladder, a bile duct, abscess, and a gastrointestinal tract, for example, drainage accumulated in a gallbladder 11 (see FIG. 8), to outside of the tissue. The drainage tube 1 is used by being inserted into an endoscope 100 (see FIGS. 6 and 8). In the present embodiment, the gallbladder 11 described above will be described below as a tissue.

As shown in FIGS. 1 and 2, the drainage tube 1 includes a front-stage tube 2 and a rear-stage tube 3. The front-stage tube 2 is to be inserted into the gallbladder 11 (see FIG. 8). The rear-stage tube 3 is joined to an end portion (hereinafter, referred to as a "first joining end portion 2b") on a side opposite to an end portion (hereinafter, referred to as a "tip end portion 2a") to be inserted into the gallbladder 11 between both ends of the front-stage tube 2. The front-stage tube 2 and the rear-stage tube 3 are joined by fitting an inner surface 3c of the rear-stage tube 3 into an outer surface 2c of the front-stage tube 2. The drainage tube 1 is provided with an operation portion 6 coupled to a base end portion 3b of the rear-stage tube 3. The "joining" between the front-stage tube 2 and the rear-stage tube 3 includes a case of engaging to a detachable degree, and further is not limited to a state where an entire joined portion between the front-stage tube 2 and the rear-stage tube 3 is in a close contact state, and also includes a joined state where a partially non-contact space is present.

### [Front-Stage Tube 2]

The front-stage tube 2 is a short tube. The front-stage tube 2 has a length approximately the same as that of an internal fistula tube (internal fistula stent) used for a general internal fistula. Specifically, the front-stage tube 2 has a length that can cause the first joining end portion 2b of the front-stage tube 2 to protrude in a stomach or a duodenum 12 near the gallbladder 11 when the front-stage tube 2 is disposed in the gallbladder 11 (see FIG. 8) . The length of the front-stage tube 2 is not particularly limited, but from the above viewpoint, the length is usually 100 mm or more, preferably 120 mm or more, and is usually 200 mm or less, preferably 150 mm or less.

For example, a thermoplastic resin such as polyethylene (PE) or polypropylene (PP) can be adopted as a material of the front-stage tube 2. It is preferable that an outer diameter and an inner diameter of the front-stage tube 2 are set to have such a size that drainage such as pus accumulated in the gallbladder can be discharged when the front-stage tube 2 is inserted into the gallbladder 11. The outer diameter of the front-stage tube 2 is usually 1 mm or more, preferably 1.5 mm or more, and is usually 3 mm or less, preferably 2 mm or less. More specifically, the outer diameter of the front-stage tube 2 is, for example, formed to be about 1.66 mm (5 Fr). From a viewpoint of discharging the drainage such as pus and a viewpoint of ensuring a mechanical strength of the front-stage tube, the inner diameter of the front-stage tube 2 is usually 0.8 mm or more, preferably 1 mm or more, and is usually 2.5 mm or less, preferably 2 mm or less.

A material that is made of any of the materials described above and which can memorize a shape can be adopted as the front-stage tube 2. For example, when the front-stage tube 2 is curved (see a front-stage tube 2F shown in FIG. 3F), the front-stage tube 2 extends in a linear shape in a state where an energization wire 4 to be described later is inserted, and the energization wire 4 passes through an organ wall of the gallbladder 11 in the state. The linearized front-stage tube 2 returns to an original shape by pulling out the energization wire 4 from the front-stage tube 2.

The front-stage tube 2 and the rear-stage tube 3 may be joined by fitting the inner surface 3c of the rear-stage tube 3 into the outer surface 2c of the front-stage tube 2. The outer diameter of the front-stage tube 2 is slightly larger than an inner diameter of a second joining end portion 3a of the rear-stage tube 3. The outer diameter of the front-stage tube 2 is smaller than an outer diameter of the rear-stage tube 3. The inner diameter of the front-stage tube 2 is smaller than an inner diameter of the rear-stage tube 3. The front-stage tube 2 is joined by a frictional force generated when the first joining end portion 2b is pressed into and fitted to the second joining end portion 3a of the rear-stage tube 3. The invention is not limited to such an embodiment. The outer surface 2c of the front-stage tube 2 and the inner surface 3c of the rear-stage tube 3 may be joined to each other by a constant joining force by using a frictional force or the like. In the present embodiment, a combination of the materials of the front-stage tube 2 and the rear-stage tube 3 is appropriately selected, whereby the outer diameter of the front-stage tube 2 and the inner diameter of the rear-stage tube 3 can be made substantially the same. Further, by an attempt to, for example, use a material that expands at a temperature in the body as the front-stage tube 2, the outer diameter of the front-stage tube 2 can also be made smaller than the inner diameter of the rear-stage tube 3.

The inner diameter of the front-stage tube 2 has a size that causes the energization wire 4 to be described later to be inserted therethrough. It is desirable that the inner diameter of the front-stage tube 2 is a diameter that can cause bile to flow smoothly.

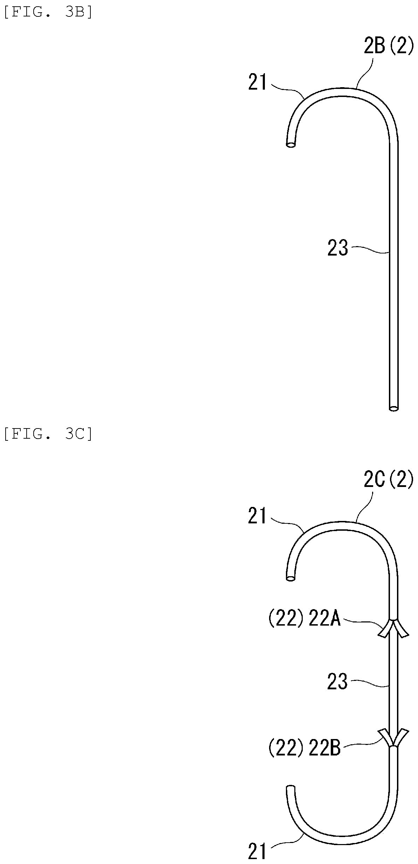

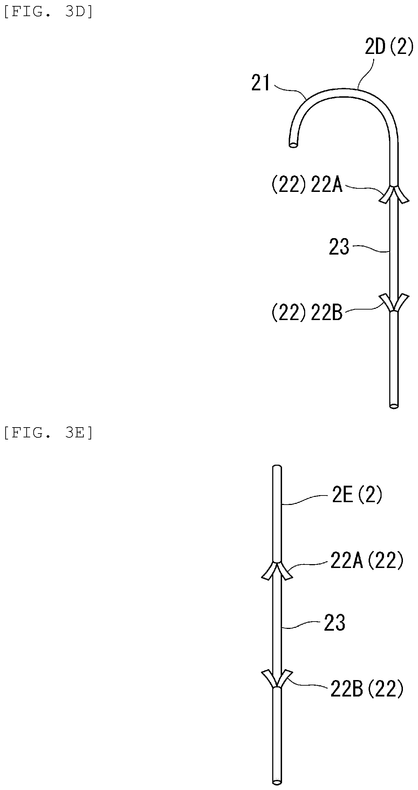

The front-stage tube 2 has a function of being able to be inserted into the gallbladder 11 and discharge the drainage such as pus. A shape of the front-stage tube 2 is not particularly limited, and for example, shapes as shown in FIGS. 3A to 3F can be adopted. Front-stage tubes 2A, 2B, 2C, and 2D shown in FIGS. 3A to 3D each include a curved portion 21 in one orientation or both orientations of an axial direction. Such a curved portion 21 is provided, whereby it is easy to prevent the front-stage tube 2 placed in the body from deviating or getting lost. In front-stage tubes 2C, 2D, 2E, and 2F shown in FIGS. 3C to 3F, a shaft portion 23 of the front-stage tube 2 is provided with a first folded portion 22A (22) to be locked when pulling is performed in a direction (a lower side in FIG. 3) opposite to an insertion direction in the axial direction of the drainage tube 1 so that the placed front-stage tube 2 does not deviate and a second folded portion 22B (22) folded in a direction opposite to the first folded portion 22A in the axial direction. When the front-stage tube 2 is inserted into the gallbladder 11, the folded portion 22A folded in a direction of an organ wall (a mucosal layer, a muscularis propria layer, a subserosa, or a serosa in a case of the gallbladder 11 in the present embodiment) of a tissue where the front-stage tube 2 is inserted, or the folded portion 22B folded in a direction of an organ wall of another tissue (a stomach or the duodenum 12) where an end portion (first joining end portion 2b) of the front-stage tube 2 opposite to the folded portion 22A is located is provided, whereby it is easy to prevent the front-stage tube 2 disposed in the body from deviating regardless of a tube shape.

The front-stage tube 2F shown in FIG. 3F has a shape that further reliably prevents the deviation by winding a tip end 23a of the shaft portion 23 in a soft-serve ice cream shape. In the front-stage tube 2F in this case, the tip end 23a in the soft-serve ice cream shape extends in a linear shape in the state where the energization wire 4 to be described later is inserted through the front-stage tube 2F, and the energization wire 4 passes through the organ wall of the gallbladder 11 in the state. When the energization wire 4 is pulled out from the front-stage tube 2F, the linearized tip end 23a of the front-stage tube 2F returns to the original shape wound in a soft-serve ice cream shape.

### [Rear-Stage Tube 3]

As shown in FIGS. 1 and 2, the rear-stage tube 3 is a tube longer than the front-stage tube 2. The rear-stage tube 3 may have a structure equivalent to that of a general external fistula tube. Specifically, the rear-stage tube 3 includes a portion where the front-stage tube 2 is fitted at a constant length (hereinafter, referred to as the "second joining end portion 3a"). A length of the second joining end portion 3a is determined in consideration of the joining force between the front-stage tube 2 and the rear-stage tube 3 and ease of separation later, and is usually set to 2 mm or more, preferably 3 mm or more, and is usually set to 20 mm or less, preferably 10 mm or less, more preferably 7 mm or less. Generally, the length may be about 5 mm.

The outer diameter of the front-stage tube 2 is slightly larger than the inner diameter of the rear-stage tube 3. The first joining end portion 2b of the front-stage tube 2 is fitted into the second joining end portion 3a. In the state where the first joining end portion 2b of the front-stage tube 2 is fitted, the second joining end portion 3a has a diameter larger than an inner diameter of the other portion (hereinafter, referred to as a "main body portion 31") of the rear-stage tube 3. As described above, the invention is not limited to such an aspect.

The length of the rear-stage tube 3 is not particularly limited, and may be usually 1400 mm or more, preferably 1600 mm or more, and may be 3000 mm or less, preferably 1800 mm or less. More specifically, for the rear-stage tube 3, for example, a tube about 1600 mm to 1700 mm is adopted. Further, the outer diameter and the inner diameter of the rear-stage tube 3 are set in consideration of the outer diameter of the front-stage tube 2, ease of insertion into the body, and ease of insertion of the energization wire 4 and a first extrusion member 5 to be described later. The outer diameter of the rear-stage tube 3 is usually 1.5 mm or more, preferably 2 mm or more, more preferably 2.3 mm or more, and is usually 4 mm or less, preferably 3 mm or less. The inner diameter of the rear-stage tube 3 is usually 1.0 mm or more, preferably 1.3 mm or more, and is usually 3 mm or less, preferably 1.5 mm or less. For example, the outer diameter of the rear-stage tube 3 can be about 2.33 mm (7 Fr).

For example, a thermoplastic resin or a thermosetting resin such as polyethylene (PE), polypropylene (PP), or polyurethane (PU) can be adopted as a material of the rear-stage tube 3. The material of the rear-stage tube 3 can be selected in relation to the material of the front-stage tube 2. For example, when polyethylene is used for both the front-stage tube 2 and the rear-stage tube 3, a combination of low-density polyethylene (LDPE) for the front-stage tube 2 and high-density polyethylene (HDPE) having excellent rigidity for the rear-stage tube 3 can be used.

The outer diameter of the front-stage tube 2 is slightly larger than an inner diameter of the second joining end portion 3a of the rear-stage tube 3. When the first joining end portion 2b of the front-stage tube 2 is pressed into and fitted to the second joining end portion 3a of the rear-stage tube 3, the front-stage tube 2 is joined to the rear-stage tube 3 by a frictional force. As described above, the invention is not limited to such an aspect.

A shape of the joined portion between the first joining end portion 2b of the front-stage tube 2 and the second joining end portion 3a of the rear-stage tube 3 is not particularly limited. For example, when the first joining end portion 2b of the front-stage tube 2 is fitted as shown in FIG. 2, the second joining end portion 3a of the rear-stage tube 3 may have a shape in which a diameter is expanded outward in a radial direction by elastic deformation. Further, for example, the inner surface 3c of the second joining end portion 3a of the rear-stage tube 3 may have a tapered shape whose diameter gradually increases toward a tip end.

In the present embodiment, the tip end portion 2a of the front-stage tube 2 and a tip end portion of the second joining end portion 3a of the rear-stage tube 3 are subjected to an R processing in order not to damage a tissue, or to pass through a wall smoothly. The tip end portion of the second joining end portion 3a is not limited to being rounded.

### [Energization Wire 4]

As shown in FIG. 4, the energization wire 4 can be inserted through the front-stage tube 2 and the rear-stage tube 3 of the drainage tube 1. The energization wire 4 is used when burning and passing through the organ wall of the gallbladder 11. The energization wire 4 has an outer diameter smaller than the inner diameter of the front-stage tube 2. The energization wire 4 forms a through hole portion 10a in the gallbladder 11 (see FIG. 8) by Joule heat generated at a tip end portion 4a of the energization wire 4 by causing a high-frequency current to flow. A base end portion 4b of the energization wire 4 is connected to a high-frequency current generation device 42 via a high-frequency power supply cord 41.

### [First Extrusion Member 5]

As shown in FIG. 5, the first extrusion member 5 for separating the front-stage tube 2 from the rear-stage tube 3 can be inserted through inside of the rear-stage tube 3 of the drainage tube 1. The first extrusion member 5 extrudes the first joining end portion 2b in a direction opposite to the tip end portion 2a to be inserted into the gallbladder 11 between both the ends of the front-stage tube 2 from the second joining end portion 3a of the rear-stage tube 3. The first extrusion member 5 can be inserted through the inside of the rear-stage tube 3.

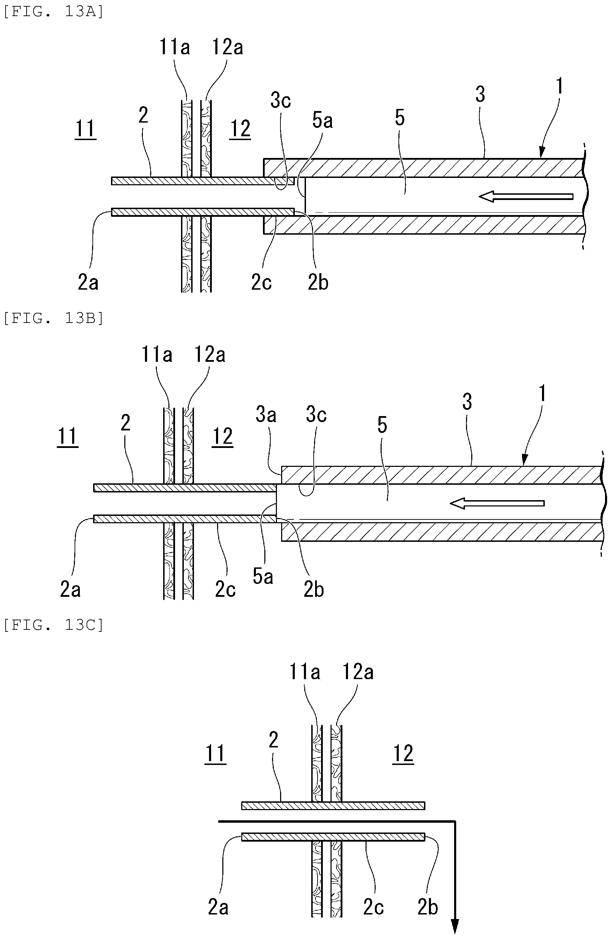

The first extrusion member 5 is not hollow (see FIG. 13). The first extrusion member 5 may be hollow in consideration of ease of handling during the extrusion, rigidity, toughness, and the like. For example, as the first extrusion member 5, a tube-shaped member may be used, or a coil-shaped member may be used. In this case, as an outer diameter of the first extrusion member 5, one at least larger than the inner diameter of the front-stage tube 2 and smaller than the inner diameter of the rear-stage tube 3 is adopted. As a material of the first extrusion member 5, for example, stainless steel, nickel titanium, or plastic can be adopted. The outer diameter of the first extrusion member 5 is set to such a size that the front-stage tube 2 can be extruded. The outer diameter of the first extrusion member 5 is usually 1 mm or more, preferably 1.2 mm or more, and is usually 2 mm or less, preferably 1.7 mm or less. More specifically, the outer diameter of the first extrusion member 5 is, for example, about 1.33 mm (4.0 Fr) to 1.5 mm (4.5 Fr). The first extrusion member 5 can also be selected based on good sliding of the front-stage tube 2 with respect to the rear-stage tube 3.

As described above, the first joining end portion 2b of the front-stage tube 2 is pressed into the second joining end portion 3a of the rear-stage tube 3 and is joined thereto by the frictional force. An extrusion tip end portion 5a located at a tip end of the first extrusion member 5 is provided in the rear-stage tube 3 such that the extrusion tip end portion 5a abut against the first joining end portion 2b of the front-stage tube 2 and can further press the first joining end portion 2b. The first extrusion member 5 can apply a pressing force larger than the frictional force generated between the front-stage tube 2 and the rear-stage tube 3 to the first joining end portion 2b of the front-stage tube 2.

### [Operation Portion 6]

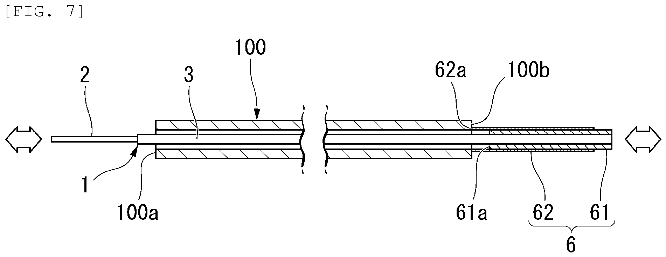

As shown in FIGS. 1, 6, and 7, the operation portion 6 has a double pipe structure including an inner pipe 61 and an outer pipe 62. Inside the outer pipe 62, the inner pipe 61 is inserted in a state of being able to be moved in a front-rear direction. Inside the inner pipe 61, the base end portion 3b of the rear-stage tube 3 is connected in a state of being inserted. A tip end portion 62a of the outer pipe 62 is disposed at a rear end portion 100b of the endoscope 100. The operation portion 6 is not disposed inside the endoscope 100. Inside the endoscope 100, the rear-stage tube 3 and the front-stage tube 2 are inserted, and the inner pipe 61 is moved with respect to the outer pipe 62 in a front-rear direction, whereby the rear-stage tube 3 connected to the inner pipe 61 is moved together with the front-stage tube 2 in the front-rear direction. When the inner pipe 61 is not inserted through the outer pipe 62 in a state where the drainage tube 1 is inserted inside the endoscope 100, as shown in FIG. 6, a state is established in which the front-stage tube 2 does not protrude from a tip end portion 100a of the endoscope 100. On the other hand, when the inner pipe 61 is inserted through the outer pipe 62 in a state where the drainage tube 1 is inserted inside the endoscope 100, as shown in FIG. 7, a state is established in which at least the front-stage tube 2 protrudes forward from the tip end portion 100a of the endoscope 100.

As shown in FIG. 6, the endoscope 100 is a well-known flexible endoscope. The endoscope 100 includes an insertion portion inserted from a tip end into the body. The endoscope 100 is used with the drainage tube 1 inserted thereto. The endoscope 100 is a medical device directly or indirectly inserted into a tissue (the gallbladder 11 in the present embodiment) in the body. An imaging unit is provided in the tip end portion 100a of the endoscope 100, and can image a portion to be treated. For example, an endoscope including optical imaging elements such as a CCD and a CMOS in the imaging unit, and an ultrasonic endoscope provided with an ultrasonic element that can scan a back side of the organ wall in addition to the imaging elements can be adopted as the endoscope.

### [Method for Discharging Pus Using Drainage Tube 1]

Next, a work procedure of drainage for discharging the pus in the gallbladder 11 by using the drainage tube 1 will be described in detail as an example based on FIGS. 6 to 13.

First, the endoscope 100 is inserted from a nose or a mouth in a state where the drainage tube 1 is introduced, and the tip end portion 100a of the endoscope 100 is disposed near the gallbladder 11 in the stomach or the duodenum 12.

Next, as shown in FIG. 6, the rear-stage tube 3 to which the front-stage tube 2 is joined is inserted through the endoscope 100. As shown in FIG. 7, when the inner pipe 61 of the operation portion 6 is moved forward inside the outer pipe 62, at least the tip end portion 2a of the front-stage tube 2 protrudes forward from the tip end portion 100a of the endoscope 100.

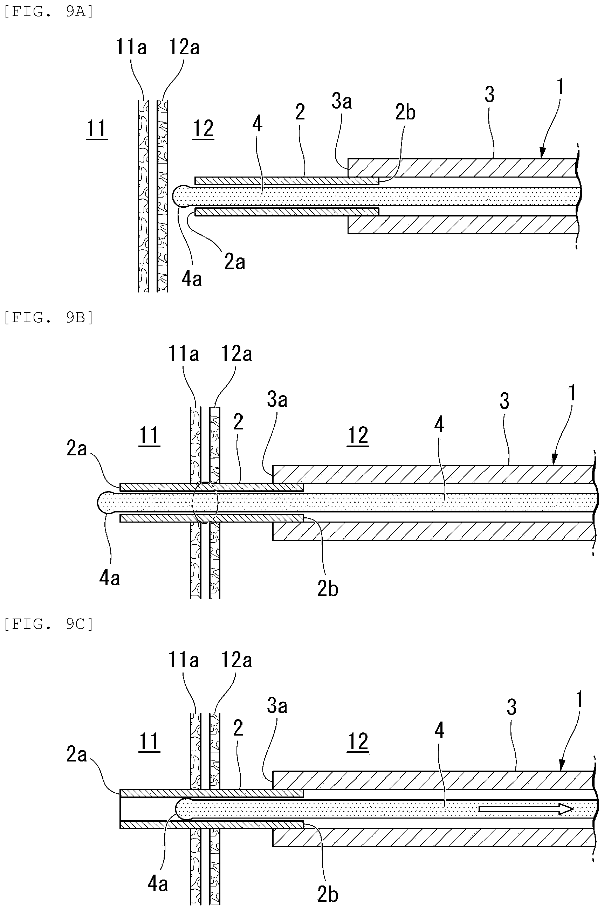

Next, as shown in FIG. 8, the energization wire 4 through which the high-frequency current flows is inserted from a rear side of the rear-stage tube 3 of the drainage tube 1, and the tip end portion 4a of the energization wire 4 burns and passes through organ walls of the stomach or the duodenum 12 and the gallbladder 11. Specifically, as shown in FIG. 9A, the tip end portion 2a of the front-stage tube 2 is disposed near an inner wall portion 12b inside the stomach or the duodenum 12. Subsequently, as shown in FIG. 9B, while the tip end portion 4a of the energization wire 4 being caused to protrude forward in the drainage tube 1 to pass through organ walls 12a and 11a of the stomach or the duodenum 12 and the gallbladder 11, the front-stage tube 2 is located in a manner of crossing both pass-through portions of the stomach or the duodenum 12 and the gallbladder 11 through which the front-stage tube 2 passes. Thereafter, as shown in FIG. 9C, the energization wire 4 is pulled out from the rear end portion 100b (see FIGS. 6 and 7) of the endoscope 100.

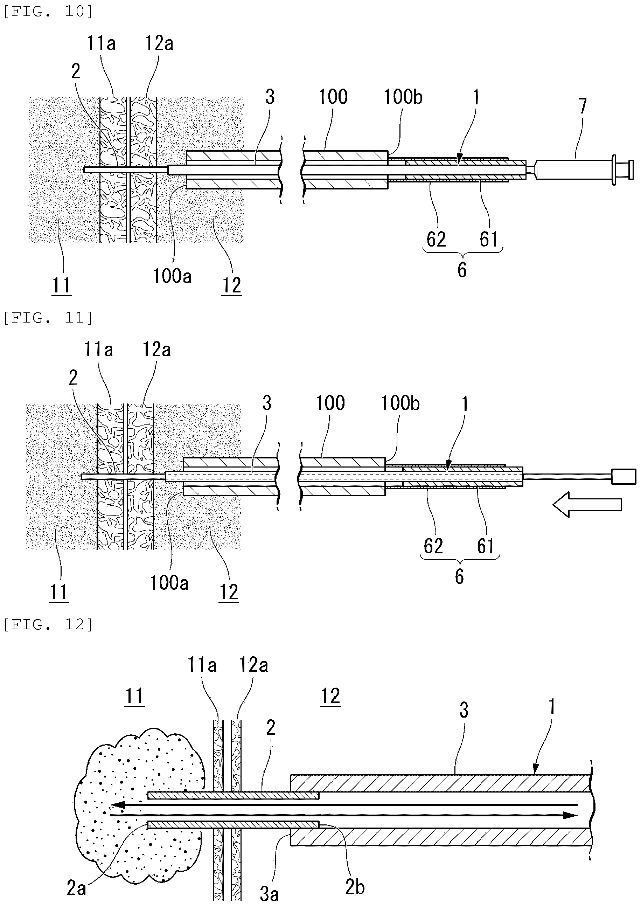

Next, as shown in FIG. 10, for example, a cleaning liquid made of a diluent such as physiological saline solution is injected into the gallbladder 11 via the front-stage tube 2 by using an injector 7 such as a syringe from the inner pipe 61 of the operation portion 6 of the drainage tube 1, the pus in the gallbladder 11 is diluted, and a mixture of the pus and the diluent is discharged to outside of the body. As shown in FIG. 11, after the discharging operation is repeated at least one time, the front-stage tube 2 disposed over the organ walls 12a and 11a of the stomach or the duodenum 12 and the gallbladder 11 is separated from the rear-stage tube 3. Depending on a state of discharge such as the pus in the gallbladder 11, the operation of injecting the diluent and discharging the mixture of the pus and the diluent to the outside of the body may not be performed.

Specifically, as shown in FIG. 12, when the cleaning liquid is injected into the gallbladder 11 by the injector 7, the pus in the gallbladder 11 is diluted. The mixture of the cleaning liquid and the pus is led out to the outside of the body through the drainage tube 1 inserted into the body. The injection of the diluent and the leading out of the mixture to the outside of the body can be performed one time or more.

As shown in FIGS. 13A to 13C, at an appropriate timing at which the discharging work is ended by diluting the cleaning liquid injected into the gallbladder 11 with the pus, the front-stage tube 2 is cut off from the rear-stage tube 3, and only the rear-stage tube 3 is taken out from inside of the body. Specifically, as shown in FIG. 13A, the first extrusion member 5 is inserted from the inner pipe 61 of the operation portion 6 into the rear-stage tube 3. The extrusion tip end portion 5a of the first extrusion member 5 abuts against the first joining end portion 2b of the front-stage tube 2 in the rear-stage tube 3, and as shown in FIG. 13B, the first extrusion member 5 is further pressed forward. In this way, when a predetermined pressing force is given to the first extrusion member 5, the first joining end portion 2b of the front-stage tube 2 exceeds the frictional force with the second joining end portion 3a of the rear-stage tube 3, and the front-stage tube 2 is detached from the rear-stage tube 3. During the operation of pressing the first extrusion member 5, the rear-stage tube 3 may be pulled rearward. Accordingly, it is possible to easily perform the detachment. When such a work procedure is performed, the work of disposing the front-stage tube 2 of the drainage tube 1 at a predetermined position in the body is completed.

Thereafter, for example, a lead-out tube (not shown) is inserted from the nose into the body, and bile in the gallbladder 11 is discharged to the outside of the body through the front-stage tube 2 and the lead-out tube.

As described above, the drainage tube 1 according to the present embodiment discharges the drainage such as the pus accumulated in the gallbladder 11 in the body to outside of the gallbladder 11. The drainage tube 1 includes the front-stage tube 2 to be inserted into the gallbladder 11 and the rear-stage tube 3 joined to the end portion on the side opposite to the end portion to be inserted into the gallbladder 11 between both the ends of the front-stage tube 2. The front-stage tube 2 and the rear-stage tube 3 are joined to each other by fitting the inner surface 3c of the rear-stage tube 3 to the outer surface 2c of the front-stage tube 2.

In the drainage tube 1 according to the present embodiment, the first joining end portion 2b of the front-stage tube 2 is joined to the second joining end portion 3a of the rear-stage tube 3 in a state of being fitted by the frictional force. Therefore, since the joining between the front-stage tube 2 and the rear-stage tube 3 can be mechanically released, it is possible to separate the front-stage tube 2 from the rear-stage tube 3 at an appropriate timing, and to perform arrangement at a predetermined position in the body while adopting a simple structure. In the present embodiment, the separation timing is different from that in a related-art case where a material that can be melted is used at a coupling portion of the two tubes including the front-stage tube 2 and the rear-stage tube 3. That is, in the present embodiment, it is possible to freely control the timing of the separation between the front-stage tube 2 and the rear-stage tube 3, and to optimally and efficiently cope with progress in treatment.

The first extrusion member 5 used to separate the front-stage tube 2 from the rear-stage tube 3 can be inserted through inside of the rear-stage tube 3 of the drainage tube 1 according to the present embodiment. The first extrusion member 5 can extrude the end portion on the side opposite to the end portion to be inserted into the gallbladder 11 between both the ends of the front-stage tube 2.

In this case, at any timing after the front-stage tube 2 is disposed at a predetermined position of an organ in the body, the first extrusion member 5 can be inserted from the rear side of the rear-stage tube 3. Accordingly, the extrusion tip end portion 5a of the first extrusion member 5 abuts against the first joining end portion 2b of the front-stage tube 2 in the rear-stage tube 3, and a predetermined pressing force is given to the first extrusion member 5 by pressing the first extrusion member 5 forward. Accordingly, in the present embodiment, the first joining end portion 2b of the front-stage tube 2 exceeds the frictional force with the second joining end portion 3a of the rear-stage tube 3, and the front-stage tube 2 can be detached mechanically from the rear-stage tube 3.

The energization wire 4 according to the present embodiment has the outer diameter smaller than the inner diameter of the front-stage tube 2. The energization wire 4, which can form a through hole in the gallbladder 11 with the tip end thereof by causing the high-frequency current to flow, can be inserted through the front-stage tube 2 and the rear-stage tube 3.

In this case, the energization wire 4 is used, the energization wire 4 can be inserted through the rear-stage tube 3 and the front-stage tube 2 of the drainage tube 1, and the gallbladder 11 can be burnt and the through hole can be formed by the tip end portion of the energization wire that protrudes from the tip end portion of the front-stage tube 2.

In the drainage tube 1 according to the present embodiment, for a tissue in the body, that is, one or more tissues (the gallbladder 11 in the present embodiment) selected from the gallbladder, the bile duct, the abscess, and the gastrointestinal tract, the front-stage tube 2 can be disposed at the tissue in the body at any timing.

In the drainage tube 1 according to the present embodiment, the rear-stage tube 3 is led out to the outside of the body. In the drainage tube 1, after the diluent is injected from the outside of the body into the gallbladder 11 in the body via the rear-stage tube 3 and the front-stage tube 2 and is mixed with the drainage, the mixture of the drainage and the diluent can be led out to the outside of the body via the front-stage tube 2 and the rear-stage tube 3.

In the present embodiment, at a stage before the front-stage tube 2 is separated from the rear-stage tube 3, and after the diluent is injected from the outside of the body into the gallbladder 11 in the body via the rear-stage tube 3 and the front-stage tube 2 and is mixed with the drainage, the mixture of the drainage and the diluent can be led out to the outside of the body via the front-stage tube 2 and the rear-stage tube 3. In the present embodiment, since the separation timing of the front-stage tube 2 can be set freely, sufficient dilution using the diluent can be performed according to a state of a patient.

In the drainage tube 1 according to the present embodiment, after the injection of the diluent and the leading out of the mixture to the outside of the body are performed one time or more, the rear-stage tube 3 can be separated from the front-stage tube 2.

In the present embodiment, at the stage before the front-stage tube 2 is separated from the rear-stage tube 3, the injection of the diluent and the leading out of the mixture to the outside of the body can be performed one time or more according to a state of the patient.

According to the drainage tube 1 in the present embodiment, it is possible to control the timing of the separation between the rear-stage tube and the front-stage tube.

The preferred embodiment of the invention has been described above, but the invention is not limited to the embodiment, modifications, and examples. In a scope not departing from the gist of the invention, additions, omissions, replacements, and other changes of the configuration can be made.

Further, the invention is not limited to the above description, and is limited only by the appended claims.

In the present embodiment, a configuration is formed in which the first extrusion member 5 used to separate the front-stage tube 2 from the rear-stage tube 3 is provided, but such a first extrusion member 5 can also be omitted. As in the embodiment described above, a configuration is formed in which the first extrusion member 5 can extrude the first joining end portion 2b on the side opposite to the tip end portion 2a to be inserted into the gallbladder 11 between both the ends of the front-stage tube 2, and can be inserted through the inside of the rear-stage tube 3, but the invention is not limited thereto. In short, it is sufficient that the front-stage tube 2 and the rear-stage tube 3 joined by fitting are provided such that the joined portion can be released at any timing.

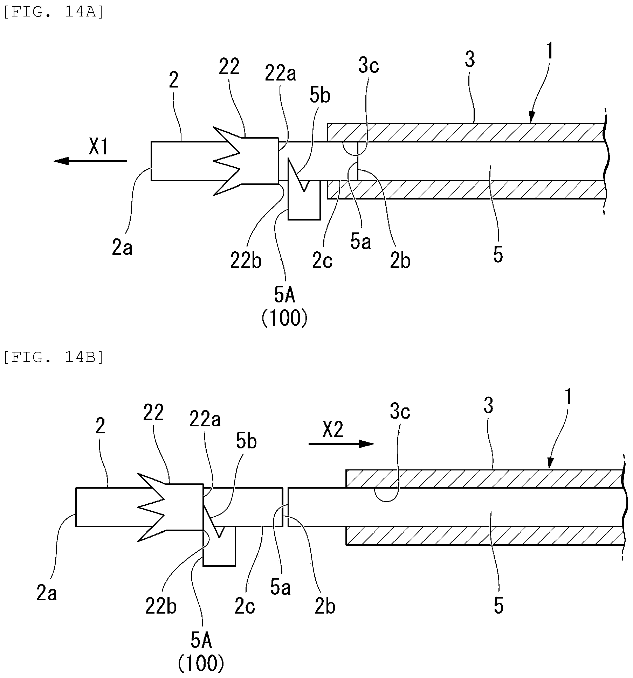

FIGS. 14A and 14B show a configuration of a second extrusion member 5A according to a modification, and are diagrams showing a state where the front-stage tube 2 is separated from the rear-stage tube 3. The modification is an example in which the first extrusion member 5 described above and the second extrusion member 5A are used in combination.

The front-stage tube 2 includes the above-described second folded portion 22B (22) shown in FIGS. 3D and 3E. A step portion 22b having a diameter larger than that of the outer surface 2c (peripheral surface) of the front-stage tube 2 is formed at a base end 22a of the second folded portion 22B. The second extrusion member 5A according to the modification is provided at the tip end of the endoscope 100. The second extrusion member 5A is a member formed with a V-shaped groove portion 5b for hooking a device (not shown) used for the endoscope 10, and can move in an axial direction (longitudinal direction) of the front-stage tube 2. The second extrusion member 5A can press the V-shaped groove portion 5b against the outer surface 2c of the front-stage tube 2. In the modification, as shown in FIG. 14A, the first joining end portion 2b of the front-stage tube 2 is pressed toward a front side X1 by the extrusion tip end portion 5a of the first extrusion member 5 inserted into the rear-stage tube 3. As shown in FIG. 14B, simultaneously with or after the pressing by the first extrusion member 5, the V-shaped groove portion 5b of the second extrusion member 5A is pressed against the outer surface 2c of the front-stage tube 2. At this time, since a state is established in which the second extrusion member 5A is locked to the step portion 22b of the second folded portion 22B of the front-stage tube 2, movement of the front-stage tube 2 toward a rear side X2 is restricted. When the rear-stage tube 3 is pulled toward the rear side X2, the front-stage tube 2 can be detached from the rear-stage tube 3.

In the modification, the first extrusion member 5 and the second extrusion member 5A are used in combination, but a method may be adopted in which only second extrusion member 5A is used without using the first extrusion member 5.

The invention is not limited to the configuration in which the energization wire 4 that has the outer diameter smaller than the inner diameter of the front-stage tube 2 and that can form the through hole in the gallbladder 11 with the tip end thereof by causing the high-frequency current to flow is provided, and the energization wire 4 can be inserted through the front-stage tube 2 and the rear-stage tube 3.

In the present embodiment, the configuration is formed in which the rear-stage tube 3 is led out to the outside of the body, and after the diluent is injected from the outside of the body into the gallbladder 11 (tissue) in the body via the rear-stage tube 3 and the front-stage tube 2 and is mixed with the drainage, the mixture of the drainage and the diluent can be led out to the outside of the body via the front-stage tube 2 and the rear-stage tube 3, but the invention is not limited thereto.

### Industrial Applicability

The drainage tube according to the invention can apply to a drainage tube that can control a timing of separation between the rear-stage tube and the front-stage tube.

### Reference Signs List

100: endoscope
1: drainage tube
2: front-stage tube
2a: tip end portion
2b: first joining end portion
3: rear-stage tube
3a: second joining end portion
3b: base end portion
4: energization wire
5: first extrusion member (extrusion member)
5A: second extrusion member (extrusion member)
6: operation portion
11: gallbladder (tissue)
12: duodenum
61: inner pipe
62: outer pipe

## Claims

1. A drainage tube for discharging drainage such as pus accumulated in a tissue in a body to outside of the tissue, the drainage tube comprising:
a front-stage tube to be inserted into the tissue; and
a rear-stage tube joined to an end portion on a side opposite to an end portion to be inserted into the tissue between both ends of the front-stage tube, wherein
the front-stage tube and the rear-stage tube are joined to each other by fitting an inner surface of the rear-stage tube to an outer surface of the front-stage tube.

2. The drainage tube according to claim 1, wherein
an extrusion member that is used to separate the front-stage tube from the rear-stage tube, and that extrudes, from the rear-stage tube, the end portion on the side opposite to the end portion to be inserted into the tissue between both the ends of the front-stage tube is provided.

3. The drainage tube according to claim 2, wherein
the extrusion member is provided to be capable of pressing the front-stage tube in a state of being inserted through inside of the rear-stage tube.

4. The drainage tube according to claim 2, wherein
the extrusion member is provided so that the rear-stage tube is movable in a direction away from the front-stage tube in a state where the extrusion member is locked to a peripheral surface of the front-stage tube.

5. The drainage tube according to any one of claims 1 to 4, wherein
an energization wire having an outer diameter smaller than an inner diameter of the front-stage tube and configured to form a through hole in the tissue with a tip end thereof by causing a high-frequency current to flow is inserted through the front-stage tube and the rear-stage tube.

6. The drainage tube according to any one of claims 1 to 4, wherein
the tissue in the body is one or more tissues selected from a gallbladder, a bile duct, an abscess, and a gastrointestinal tract.

7. The drainage tube according to any one of claims 1 to 4, wherein
the rear-stage tube is led out to outside of the body, and after a diluent is injected from outside of the body into the tissue in the body via the rear-stage tube and the front-stage tube and is mixed with the drainage, a mixture of the drainage and the diluent is led out to the outside of the body via the front-stage tube and the rear-stage tube.

8. The drainage tube according to claim 7, wherein
after the injection of the diluent and the leading out of the mixture to the outside of the body are performed one time or more, the rear-stage tube is separated from the front-stage tube.
